(19) Europäisches Patentamt
European Patent Office
Office européen des brevets

(11) Publication number: **0 345 789 A2**

# (12) EUROPEAN PATENT APPLICATION

(21) Application number: **89110408.5**

(51) Int. Cl.[4]: **C12N 11/00 , G01N 33/547**

(22) Date of filing: **08.06.89**

(30) Priority: **10.06.88 US 204701**

(43) Date of publication of application:
**13.12.89 Bulletin 89/50**

(84) Designated Contracting States:
**AT BE CH DE ES FR GB GR IT LI LU NL SE**

(71) Applicant: **Taylor, Keith E.**
**165 California Avenue**
**Windsor Ontario N9B 2Y4(CA)**

Applicant: **Boss, Sheila C.**
**5216 Boulevard Samson #5**
**Laval Quebec H7U 2J3(CA)**

(72) Inventor: **Taylor, Keith E.**
**165 California Avenue**
**Windsor Ontario N9B 2Y4(CA)**
Inventor: **Boss, Sheila C.**
**5216 Boulevard Samson #5**
**Laval Quebec H7U 2J3(CA)**

(74) Representative: **Patentanwälte Grünecker, Kinkeldey, Stockmair & Partner**
**Maximilianstrasse 58**
**D-8000 München 22(DE)**

## (54) Method and device for attachement of biologically useful materials to a solid phase.

(57) A method and kit for indirectly attaching a biologically useful material to a solid phase by means of a linking polymer is described. The linking polymer is selected from difunctional polyalkylene ether polymers wherein the difunctionality is at the distal and proximal ends of the polymer. The proximal end of the polymer is attached to the solid phase and the distal end of the polymer is joined to the material. Very low density of the linking polymer (less than 100 nanomoles per square meter) reduces interference with and increases the activity of the material. Enzymes and other active molecules or combinations of molecules are immobilized in the solid phase.

EP 0 345 789 A2

# IMPROVED METHOD AND DEVICE FOR ATTACHMENT OF BIOLOGICALLY USEFUL MATERIALS TO A SOLID PHASE

## BACKGROUND OF THE INVENTION

### (1) Field of the Invention

The present invention relates to a method and device wherein biologically useful materials are indirectly attached to exposed surfaces of a solid phase so that a low density of such materials is provided on the surface. In particular the present invention relates to the use of low densities of linking polymers with proximal ends covalently joined to the surface and distal ends covalently joined to the material.

### (2) Prior Art

Immobilized biomolecules have become of general utility in the last two decades in such diverse formats as particulate catalysts readily separated from a reaction mixture (K. Mosbach, ed., Methods in Enzymology, Vol. XLIV, pages ii-ix, 3-7, 317-320, 717-738 and 776-792, Academic Press, N.Y., 1976; Vol. 135, pages ii-viii and 596-604, Academic Press, N.Y., 1987; Vol. 136, pages ii-viii, 230-233 and 423-431, Academic Press, N.Y., 1987), affinity chromatography matrices (W. B. Jakoby and M. Wilchek, eds., Methods in Enzymology, Vol. XXXIV, pages ii-xi, 3-10, 242-253 and 653-670, Academic Press, N.Y., 1974), solid-phase immunochemicals (J. J. Langone and H. Van Vunakis, eds., Methods in Enzymology, Vol. 70, pages ii-vii and 334-355, Academic Press, N.Y., 1980; Vol. 73, pages ii-viii and 224-239, Academic Press, N.Y., 1981; Vol. 92, pages ii-viii and 345-359, Academic Press, N.Y., 1983) and immobilized whole cells (K. Mosbach, ed., Methods in Enzymology, Vol. 135, pages 173-175, Academic Press, N.Y., 1987; Vol. 136, pages 329-342, Academic Press, N.Y., 1987; I. Chibata and L. B. Wingard, Jr., eds., Applied Biochemistry and Bioengineering, Vol. 4, pages ii-vi and 1-9, Academic Press, N.Y., 1983). The end uses of such conjugates include fine and bulk chemicals production, food processing and waste treatment, analytical procedures (P. W. Carr and L. D. Bowers, Immobilized Enzymes in Analytical and Clinical Chemistry, pages ii-v, xi-xvii and 408-441, J. Wiley & Sons, N.Y., 1980) and detection, separation and/or purification of other molecules of biological interest.

The method of immobilization for biological macromolecules could simply be retention in soluble form behind a semi-permeable membrane but in most applications biological molecules, large or small, have been associated with a solid phase in various formats. Particles or beads, either porous or non-porous, have been used in packed column or suspension form; membranes or nets of various porosities have served; and tubing has been inner surface-derivatized with biological molecules.

Materials used for the solid phase encompass the whole range of polymers with some of the most prevalent being polymers of ethylenically-unsaturated monomers (e.g.-styrene, acrylic acid derivatives, ethylene, propylene), polysaccharides (e.g.- cellulose, dextran, agarose), polypeptides (e.g.- gelatin/collagen, cross-linked proteins such as albumin), nylon and glass.

The means of association or attachment of biological molecules to the solid phase could be by entrapment during polymerization or by electrostatic or hydrophobic adsorption to the pre-formed polymer. Both of these methods allow desorption or leaching of biological molecules from the solid phase and, therefore, the preferred means of attachment, is covalent linkage via, for example, amide, ester, ether, thioether, amine, imine or diazo functional groups. To achieve such covalent attachment it is usually necessary to activate the solid phase by introduction of appropriate reactive surface functional groups, for example, of the active ester, acid halide, isothiocyanate, cyanate, imidate, alpha-halocarbonyl, oxirane and related types. With respect to the biological macromolecules to be attached, proteins are frequently attached through their surface carboxyl, amino or tyrosyl groups; polysaccharides, glycoproteins, nucleic acids and nucleotides may be attached via carbohydrate oxidation followed by imine formation with or without simultaneous or subsequent reduction (0. R. Zaborsky and J. Ogletree, Biochem. Biophys. Res. Commun. 61,210-216 (1974); J. Christison, Chem. Ind. 215-216 (1972)). With respect to other biological molecules similar functional groups on the molecule, or introduced into it by chemical modification, are coupled with complementary functional groups on the solid phase.

In the attachment process above the advantage of a spacer arm, or linker, between solid phase and biological macromolecule has been recognized for many years (P. Cuatrecasas, J. Biol. Chem. 245, 3059-3065 (1970)). Spacer arms or linkers generally used are hydrocarbons which are approximately six methylene units in length such as 1,6-diaminohexane, adipic dihydrazide, 6-aminohexanoic acid and 3,3′-diaminopropylamine. Some use has been made of polypeptides, either natural (e.g.- albumin, gelatin) or synthetic (e.g.- poly(L-lysine) and other poly(amino acids)).

Patent activity relevant to the present invention touches on several of the aspects generally surveyed above. With respect to the linker molecules, Frey and Simmons, U.S. Patent No. 4,581,337, teach immobilization of small antigens;

a latex as a solid support of refractive index >1.54 and particle diameters in the range 0.03 to 0.1 micrometer;

polyether polyamino linkers of the general formula:

$H[NHC_2H_3R(OC_2H_3R)_xOC_2H_3R]_yNH_2$

where x = 0-70 and y = 1-20 and R = H or $CH_3$;

functional groups on the latex particles, for attachment of linkers, consisting of epoxy, carboxy or aldehyde groups; and

the use of these particles for photometric detection of particle agglutination. In the preferred embodiment, including a procedure for synthesis of the linkers, Frey and Simmons have y = 2 in the above formula. This ensures that each linker has at least one non-terminal nitrogen, that is, a nitrogen capable of acting as an ion exchanger in the pH range below ca. 9. Particles derivatized with such linkers could, therefore, adsorb molecules of biological interest by electrostatic means, in addition to the covalent means claimed by the inventors. This would not happen if y was equal to 1 which would provide a diamine. The degree of linker substitution on the particles (i.e. surface density) was not addressed in the disclosure and in addition the examples show saturation of the particles.

In European patent application No.863097572, A. L. Finkenaur discloses the use of polyether diamines (Jeffamine® ED-600 in the example) of the type used in the present invention but in such a way as to ensure surface saturation by the linker (i.e., a coating of linker is covalently attached to the surface). Furthermore, linkers of length less than 300 Angstrom units are disclosed.

With respect to solid phase activation reactions, Hornby and Morris, U.S. Patent No. 4,115,305, disclose a nylon support activated by triethyloxonium tetrafluoroborate prior to incorporation of hexamethylenediamine as linker. In this procedure, following their published protocol (Biochem. J. 147 593-603 (1975)), the surface to be derivatized is exposed to a concentrated solution of the alkylating agent (10% w/w in methylene chloride), thereby ensuring saturation of the available surface with sites of activation (imidates in this case).

Concerning the covalent immobilization of carbohydrate-containing molecules of biological interest through the carbohydrate residue(s), Quash, U.S. Patent No. 4,217,338 and continuation No. 4,419,444, discloses oxidative activation of the target molecules either by chemical or enzymatic means, followed by reaction (imine formation) with a support carrying active amino groups. The latter were introduced in a prior step from such linkers as hexamethylenediamine, adipic dihydrazide or p-hydrazinobenzoic acid. Imines resulting from the incubation of amino-containing support and oxidized glycoconjugates (e.g., gamma globulins) were reduced by sodium borohydride in some examples.

## GENERAL DESCRIPTION

The present invention relates to an improvement in a method whereby a biologically useful molecule is indirectly attached to an exposed surface of a solid phase by means of a linking polymer which is covalently connected to the molecule at a distal end of the polymer and to the solid phase at a proximal end of the polymer the improvement which comprises: providing a linear polyalkylene ether as the linking polymer covalently connected to the solid phase at the proximal end and with a chemically functional group at the distal end which can be reacted with the biologically useful molecule or contains the biologically useful molecule, wherein the polymer has a length of about 1 to 100 nanometers and wherein the linking polymer is attached to the solid phase dispersed in a low density of less than about 100 nanomoles per square meter of the exposed surface area of the solid phase.

Further the present invention relates to a method whereby a biologically useful molecule is indirectly attached to an exposed surface of a solid phase by means of a linking polymer which is covalently connected to the molecule at a distal end of the polymer and to the solid phase at a proximal end of the polymer the improvement which comprises a polyalkylene ether linking polymer selected from

$XC_2H_3R(OC_2H_3R)_nOC_2H_3RX$

wherein n is 5 to 200, R is selected from the group consisting of hydrogen or alkyl and X is selected from the group consisting of $-NH_2$, $-NHCONHNH_2$, $-NHCO(CH_2)_pSH$, $-OCO(CH_2)_pSH$, wherein p is 1 to 3, and a halogen selected from the group consisting of chlorine and bromine and from

$XCH_2(OC_2H_3R)_nOCH_2X$

wherein n is 5 to 200, R is H or alkyl and X is selected from the group consisting of $CO_2H$ or $-CONHNH_2$ which is covalently linked by reaction with one of X with the solid phase and with the other of X can be covalently linked with the biologically useful molecule or contains the biologically useful molecule, wherein the linking polymer is attached to the exposed surface of the solid phase in a density between about 10 to 100 nanomoles per square meter of the exposed surface area of the solid phase.

Further still the present invention relates to a linking polymer wherein the linking polymer is

$$H_2N\underset{CH_3}{\underset{|}{C}}HCH_2-[O\underset{CH_3}{\underset{|}{C}}HCH_2]_a-[OCH_2-CH_2]_b-[OCH_2\underset{CH_3}{\underset{|}{C}}H]_c-NH_2$$

where a plus c are 2.5

and b is between 8.5 and 131.5 and wherein the length of the linking molecule is about 50 to 650 Angstroms.

Further still the present invention relates to an improvement in a device for attaching a biologically useful molecule to a solid phase by means of a linking polymer which is covalently connected to the molecule at the distal end of the polymer and to the solid phase at the proximal end of the polymer the improvement which comprises: providing a linear polyalkylene ether as the linking polymer covalently connected to the solid phase at the proximal end and with a chemically functional group at the distal end which can be reacted with the biologically useful molecule or containing the biologically useful molecule, wherein the polymer has a length of about 1 to 100 nanometers and wherein the linking polymer is attached to the solid phase dispersed in a low density of less than about 100 nanomoles per square meter of the exposed surface area of the solid phase.

Finally the present invention relates to a process for attaching a linking polymer to a solid phase to provide a support for a biologically useful molecule which comprises: providing a linking polymer which is a polyalkylene ether linking polymer selected from

$XC_2H_3R(OC_2H_3R)_nOC_2H_3RX$

wherein n is 5 to 200, R is selected from the group consisting of hydrogen or alkyl and X is selected from the group consisting of $-NH_2$, $-NHCONHNH_2$, $-NHCO(CH_2)_pSH$, $-OCO(CH_2)_pSH$, wherein p is 1 to 3, and a halogen selected from the group consisting of chlorine and bromine and

$XCH_2(OC_2H_3R)_nOCH_2X$

wherein n is 5 to 200, R is H or alkyl and X is selected from the group consisting of $-CO_2H$ or $-CONHNH_2$ which is covalently linked by reaction with one of X with the solid phase and with the other of X which can be covalently linked with the biologically useful molecule or contains the biologically useful molecule, wherein the linking polymer is attached to the exposed surface of the solid phase in a density between about 10 to 100 nanomoles per square meter of the exposed surface area of the solid phase; reacting a solid phase with limited amounts of an activating agent in a solvent which is non-reactive with the activating agent to provide exposed groups which are reactive with X at one end of the linking polymer, wherein the exposed groups are present in a density of less than 100 nanomoles per square meter; and reacting the linking polymer with the solid phase in a solvent for the linking polymer, wherein the linking polymer is attached to the solid phase in a density of less than about 100 nanomoles per square meter.

The present invention relates to a method of immobilization of molecules of biological interest via long, hydrophilic, uncharged spacer arms (in such a way as to maximize biological activity). This entails covalent attachment of spacer arms (also known as linkers) to the solid phase of interest at surface densities of less than 100 $nmol/m^2$ and subsequent attachment of the molecule of biological interest, be it small or large, to the distal end of the spacer. In this method:

(a) the solid phase (also known as support, substrate or matrix) could be any of the following individual polymers or composites thereof: polymers and copolymers of ethylenically-unsaturated monomers (e.g.-styrene, acrylic acid and its derivatives, ethylene, propylene), polysaccharides (e.g.- cellulose, agarose, cross-linked dextrans), glass and other silicates, proteins (e.g.- gelatin, cross-linked albumin, collagen and its composites such as bone), polycarbonate, nylon. In physical form the solid phase could be porous or non-porous to molecules of biological interest.

(b) functional groups of a given type, which occur naturally or which have been introduced by chemical modification, on the solvent-exposed surfaces of the solid phase are covalently attached to the proximal end of the spacer arm molecules. Functional groups appropriate for this purpose are: amino, hydroxyl, carboxyl, aldehyde, epoxy, imidate or sulfhydryl or various activated forms of these groups. The specific functional group chosen for use here depends on the proximal spacer arm functional group to be covalently attached to it. Various condensing agents may be used in this coupling of solid phase and proximal spacer arm functional groups. Spacer arm functional group density is controlled at this stage.

(c) the spacer arm molecules employed are of the general formula:

$XC_2H_3R(OC_2H_3R)_nOC_2H_3RX$

where n = 5-200, R = H or alkyl and X = $-NH_2$, $-NHCONHNH_2$, $-NHCO(CH_2)_pSH$, $-OCO(CH_2)_pSH$ (where p = 1-3) or halogen or of the general formula:

$XCH_2(OC_2H_3R)_nOCH_2X$

where n = 5-200, R = H or alkyl and X = $-CO_2H$ or $-CONHNH_2$. These formulae include such preparations as Texaco's Jeffamine® ED-series and products from Evans Chemetics and Sigma Chemical Co.

(d) the distal functional group of the support-anchored spacer arm is covalently attached to the compound of biological interest through a functional group naturally occurring on the latter or introduced there through chemical modification. Functional groups on the molecule of biological interest appropriate for this purpose are amino, hydroxyl, carboxyl, aldehyde or sulfhydryl.

(e) a variation of the foregoing would comprise the one-step covalent attachment of two or more molecules of biological interest where those molecules, in a prior treatment, had been covalently attached to each other with or without spacer or linker molecules.

The covalent immobilization of compounds of biological interest to solid support matrices is accomplished in such a manner that their biological usefulness (activity) is not markedly compromised. These compounds may include enzymes, antibodies, antigens, enzyme substrates, coenzymes, metabolites, drugs and/or their metabolites, hormones, haptens, fluorophores, chromogens, polysaccharides, polynucleotides, whole cells, bacteria, protozoa, fungi, viruses, peptides, other proteins, toxins, lipids and derivatives thereof.

The advantages of this invention over prior art are based on the immobilized molecule's behaving like the same molecule free in solution with respect to thermodynamic properties (e.g., affinity for ligands) and kinetic behavior (e.g., rate of ligand association, disassociation, efficiency of catalysis, diffusional sampling of large volumes).

In general terms, the tactics chosen to achieve the foregoing advantages are based on appropriate choice of linker polymers and their distribution on the surface of the solid support at one end (proximal end) and the molecule of biological interest at the other end (distal end). Firstly, with respect to structure the linkers chosen are long, linear polymers, with extended lengths of hundreds to thousands of Angstrom units (10's to 100's of nm) so that the immobilized molecule is subtended out of the range of surface field effects and so that it has relatively unhindered accessibility to molecules in solution (enzyme substrate, antibody, antigen, hapten, etc.). These linkers are hydrophilic so that in aqueous solution extended conformations would be expected. Secondly, with respect to distribution on the surface of the solid support, the linkers are sparsely substituted, on the order of one for every few thousand Angstrom units squared (less than 100 $nmol/m^2$). This has several consequences: macromolecules are, on average, only attached to the solid support by one linker (i.e., single-point attachment), thus improving their diffusional mobility at the distal end while at the same time minimally perturbing the molecule's covalent structure; small molecules (e.g. substrates and coenzymes for enzymes, haptens for antibodies) can also approach the tethered molecule of biological interest from the "underside" (i.e., nearer the surface of the solid support), thus increasing diffusional accessibility; immobilized small molecules of biological interest (e.g. haptens, biotin, toxins, hormones) may be freely approached by macromolecules in the bulk solution without the latter experiencing field effects of the solid support. Lastly, with respect to the linking polymers distal attachment to molecules of biological interest, the specific type and, on macromolecules, location of the covalent connection may be controlled to minimize perturbation of biological activity. One large group of molecules of biological interest in which non-functional residues may be selected as the attachment site are glycoproteins where, for example, the carbohydrate portions are known not to be involved in enzyme or antibody activity.

The linkers used in this invention are preferably commercially available $\alpha,\omega$-bifunctional oxyethylene oligomers or oxypropylene-capped oxyethylene oligomers. A small variety of terminal functional groups, both the same, are available in the commercial products and from these many others are available by simple chemical reactions. The choice of linker functional group will be based on the coupling reaction to be performed at the proximal (solid support) and distal (molecule of biological interest) ends. Linkers of the

α,ω-diaminopoly(oxyalkylene)type with molecular weight around 600 Daltons (extended length ca. 60 Å) have been used (K. E. Taylor and L. Chen, abstract Biol-22, 61st Canadian Chemical Conference, Winnipeg, 4-7 June, 1978; K. E. Taylor, L. Chen and K. Wu, abstract 390, 27th IUPAC Congress, Helsinki, 27-31 August, 1979). However, it was not appreciated by us until much later that longer linking polymers at low density could substantially improve the performance of distally-attached biological macromolecules (See Example 4).

The solid supports of this invention must be activated for attachment of the linking polymer and it is at this stage that the surface density of linkers can be controlled by providing limiting amounts of an extremely reactive reagent under mild conditions. An example of this approach is provided in Example 1 where nylon is the support material and a triethyloxonium salt is the activating agent. The resulting imidate sites of activation on the nylon are subsequently reacted with an excess of α,ω-diamino linking polymer. The same approach would serve for materials such as polyacrylamide and its copolymers. Alternatively, a more inert support could first be converted to an intermediate derivative of medium to high functional group density under more vigorous conditions and then, subsequently, the intermediate derivative could be exposed to a limiting amount of activating agent prior to linker attachment. This approach serves for polystyrene which, via the intermediate amino form (J.A.G. Aleixo et al., J. Immunoassay 6, 391-407 (1985)), is treated with an acylating agent such as carbonyl diimidazole (G.S. Bethell, J.S. Ayers, W.S. Hancock and M.T.W. Hearn, J. Biol. Chem. 254, 2572-2574 (1979)) in limiting amounts followed by an appropriate α,ω-bifunctional linker. Lastly, limited functionalization prior to linker incorporation could be achieved by appropriate copolymer formulation (e.g. styrene-acrylic acid and related mixtures).

Attachment of the molecule of biological interest to the distal linking polymer functional group is carried out by means known from the prior art. For example, if a glycoprotein is to be attached, then the periodate- or galactose oxidase- or analogously-oxidized glycoprotein is incubated with amino- or, preferably, acyl hydrazido-terminated linking molecule in an appropriate buffer to allow Schiff base formation (imine or hydrazone) which may or may not be stabilized by subsequent borohydride treatment or concomittant cyanoborohydride reduction. If attachment is not to be through carbohydrate, the range of conventional peptide coupling methods could be employed. For example, water-soluble carbodiimides and related reagents have been frequently used in the preparation of affinity chromatography matrices. Alternatively, directed thiol-disulfide interchange reactions could provide the means of attachment as in covalent chromatography (K. Brocklehurst, J. Carlsson, P. J. Kierstan and E. M. Crook, Biochem. J. 133, 573-584 (1973)). In this approach thiol groups would be those naturally occurring on the compound of biological interest or thiols introduced there by chemical modification using either an amino group-specific reagent (e.g. as in J. Carlsson, H. Drevin and R. Axen, Biochem. J. 173, 723-737 (1978)) or a carbohydrate-specific reagent (e.g. as in K. E. Taylor and Y.-C. Wu, Biochem. Int. 1, 353-358 (1980)).

## SPECIFIC DESCRIPTION

### Example 1

**Nylon Activation and Incorporation of Linker.**

A one meter length of nylon-6 tubing, 1 or 0.5 mm bore, (Portex, Hythe, UK) was perfused with a mixture of calcium chloride (18.6%, w/w) and water (18.6%, w/w) in methanol at 60 °C for 20 minutes. The tubing was washed with aqueous methanol then methanol and dried in vacuo over phosphorous pentoxide. The tubing was filled with a solution of triethyloxonium fluoroborate (Fluka, Buchs, Switzerland) in anhydrous methylene chloride at a concentration of approximately 15 M. The filled tubing was incubated 50 minutes at room temperature, emptied, washed with anhydrous methylene chloride and filled with a solution of an , -diamine such as Jeffamine® ED-600 (Texaco, Don Mills, Canada; 10% v/v) in dimethylformamide. Incubation was continued for 4 hours at room temperature and then the tubing was emptied and washed in turn with: dimethylformamide, water, 0.1 M hydrochloric acid, 0.1 M sodium bicarbonate, 3 M sodium chloride, 0.2 M potassium chloride and water. Thereafter the tubing was filled with 0.1 M phosphate buffer of pH 7.5 and stored at 4 °C until use.

Example 2

Incorporation of Linker into Aminopolystyrene.

Polystyrene balls (7.6 mm diameter) were nitrated and then reduced with sodium dithionite according to J.A.G. Aleixo et al., J. Immunoassay 6, 391-407 (1985). The resulting aminopolystyrene balls were treated with 1,1′-carbonyl diimidazole at 1mM (this concentration is variable, depending on the desired level of activation) in acetonitrile for 30 minutes at room temperature, then washed with the same solvent and incubated with 10% (w/v) Jeffamine® ED-600 in acetonitrile for 1 hour at room temperature. Finally the balls were washed in turn with acetonitrile, water, 0.2 M hydrochloric acid, water and 0.1 M phosphate buffer of pH 7.5.

Example 3

Glycoenzyme Oxidation and Immobilization on Derivatized Nylon.

Enzyme, either horseradish peroxidase (Boehringer, Dorval, Canada) or glucose oxidase (Sigma, St. Louis, USA), at 1-2 mg/mL in 0.1 M phosphate buffer of pH 7.5 was mixed with an equal volume of aqueous sodium metaperiodate (12 mM) and let stand 90 minutes at room temperature in the dark. The solution was then mixed with one-half volume of aqueous ethylene glycol (0.32 M) and incubated a further 60 minutes. The solution was subjected to ultrafiltration twice with 10 volumes of the same buffer (cell and membranes from Amicon Corp., Oakville, Canada; YM-30 and XM-50 membranes for peroxidase and glucose oxidase, respectively) and then made up to approximately the original concentration (concentrations determined by spectrophotometric analysis). Nylon tubing derivatized in Example 1 was filled with the enzyme solution just described and incubated 6 or 18 hours at 4°C for peroxidase or glucose oxidase, respectively. The tubing was emptied, flushed with copious amounts of the same phosphate buffer and stored filled with same. Samples of such tubing retained 92% of the respective original activities after exposure to sodium chloride at 1 M in buffer but only 20 and 11% of their original peroxidase and glucose oxidase activities, respectively, after exposure to 10 mM hydrazine in buffer. Release of the respective proteins was quantitatively verified by spectrophotometric analysis of the incubation solutions.

Example 4

Effect of Immobilization and Linker Length on Enzyme Kinetic Performance.

Soluble or immobilized horseradish peroxidase was evaluated according to the Michaelis-Menten model with hydrogen peroxide as substrate. The two derived parameters $K_m$, the Michaelis constant, and $V_{max}$, the maximum velocity, were calculated by regression analysis of initial reaction rates at various peroxide concentrations in a 0.1 M sodium phosphate buffer of pH 7.5 which also contained sodium 2-hydroxy-3,5-dichlorobenzenesulfonate at 9 mM and 4-aminoantipyrene at 2.4 mM. The reactions were monitored spectrophotometrically at 510 nm at a temperature of 25°C. The results are shown in Table 1

Table 1

| | $K_m$ | $V_{max}$[c] |
|---|---|---|
| | mM ± SD | $mM.min^{-1}mg^{-1}$± SD |
| Soluble Enzyme[a] | | |
| Native<br>Oxidised | 0.059 ± 0.005<br>0.086 ± 0.027 | 0.399 ± 0.006<br>0.271 ± 0.011 |
| Immobilized Enzyme[a,b] | | |
| Ethylenediamine<br>ED-600 [d]<br>ED-2001<br>ED-6000 | 1.060 ± 0.226<br>0.282 ± 0.063<br>0.231 ± 0.010<br>0.125 ± 0.030 | 0.366 ± 0.168<br>0.342 ± 0.045<br>0.290 ± 0.010<br>0.322 ± 0.009 |

[a]Results are the mean of duplicate experiments with hydrogen peroxide concentrations ranging from 0.016 to 0.640 mM.

[b]Nylon tubing was derivatized with the α,ω-diamines noted (lengths in the approximate range 6-650 Å) as outlined in Example 1. Ethylenediamine is used as a reference point alo g with the native and oxidized enzyme. Amine-substituted nylon was then incubated with oxidized peroxidase as outlined in Example 3.

[c]$V_{max}$ data were normalized with respect to mg protein in the reaction mixture for soluble enzyme runs. For immobilized enzymes the data were normalized with respect to t e mg protein that disappeared from the starting solution, pro-rated to the length of tubing used in the kinetic run (approximately 10 cm).

[d]Technical Bulletin, The Jeffamines, Texaco Chemical Company, Bellaire, Texas.

The results in Table 1 show that the longer the linking polymer, the more nearly the $K_m$ approaches or meets the unattached native enzyme. It is thus preferred that the linking polymer have a length between about 400 and 650 Angstroms.

## Example 5

One-Step Immobilization of Enzyme-Enzyme Clusters.

Glucose oxidase and horseradish peroxidase were separately oxidized and purified as in Example 2, except that a final periodate concentration of 1 mM was used for peroxidase. Oxidized glucose oxidase was incubated with adipic dihydrazide at 10 mM in the presence or absence of sodium cyanoborohydride at 10 mM in 0.1 M phosphate buffer of pH 7.5 for two hours at room temperature. The derivative was purified by ultrafiltration (XM-50 membrane) and then incubated with the mildly-oxidized peroxidase overnight at 4° C in the same buffer. The resultant conjugates ("clusters") were purified by ultrafiltration on a membrane of molecular weight cutoff around 300,000 Daltons (XM-300) and then they were re-oxidized, purified and immobilized on the derivatized nylon tubing of Example 1 as in Example 3.

It is intended that the foregoing description be only illustrative of the present invention and that the present invention be limited only by the hereinafter appended claims.

## Claims

-1- In a method whereby a biologically useful molecule is indirectly attached to an exposed surface of a solid phase by means of a linking polymer which is covalently connected to the molecule at a distal end of the polymer and to the solid phase at a proximal end of the polymer the improvement which comprises: providing a linear polyalkylene ether as the linking polymer covalently connected to the solid phase at the proximal end and with a chemically functional group at the distal end which can be reacted with the biologically useful molecule or contains the biologically useful molecule, wherein the polymer has a length of about 1 to 100 nanometers and wherein the linking polymer is attached to the solid phase dispersed in a low density of less than about 100 nanomoles per square meter of the exposed surface area of the solid phase.

-2- In a method whereby a biologically useful molecule is indirectly attached to an exposed surface of a solid phase by means of a linking polymer which is covalently connected to the molecule at a distal end of the polymer and to the solid phase at a proximal end of the polymer the improvement which comprises: a polyalkylene ether linking polymer selected from
$XC_2H_3R(OC_2H_3R)_nOC_2H_3RX$
wherein n is 5 to 200, R is selected from the group consisting of hydrogen or alkyl and X is selected from the group consisting of $-NH_2$, $-NHCONHNH_2$, $-NHCO(CH_2)_pSH$, $-OCO(CH_2)_pSH$, wherein p is 1 to 3, and a halogen selected from the group consisting of chlorine and bromine and from
$XCH_2(OC_2H_3R)_nOCH_2X$
wherein n is 5 to 200, R is H or alkyl and X is selected from the group consisting of $CO_2H$ or $-CONHNH_2$ which is covalently linked by reaction with one of X with the solid phase and with the other of X can be covalently linked with the biologically useful molecule or contains the biologically useful molecule, wherein the linking polymer is attached to the exposed surface of the solid phase in a density between about 10 to 100 nanomoles per square meter of the exposed surface area of the solid phase.

-3- The method of Claim 1 wherein the linking polymer is

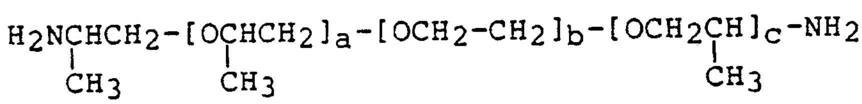

where a plus c are 2.5
and b is between 8.5 and 131.5 and wherein the length of the linking molecule is about 50 to 650 Angstroms.

-4- The method of Claim 1 including the step wherein the biologically useful molecule is covalently linked to the linking polymer.

-5- The method of Claim 2 including the step wherein the biologically useful molecule is covalently linked to the linking polymer.

-6- The method of Claim 3 including the step wherein the biologically useful molecule is covalently linked to the linking polymer.

-7- In a device for attaching a biologically useful molecule to a solid phase by means of a linking polymer which is covalently connected to the molecule at the distal end of the polymer and to the solid phase at the proximal end of the polymer the improvement which comprises:

(a) providing a linear polyalkylene ether as the linking polymer covalently connected to the solid phase at the proximal end and with a chemically functional group at the distal end which can be reacted with the biologically useful molecule or containing the biologically useful molecule, wherein the polymer has a length of about 1 to 100 nanometers and wherein the linking polymer is attached to the solid phase dispersed in a low density of less than about 100 nanomoles per square meter of the exposed surface area of the solid phase.

-8- In a device for attaching a biologically useful molecule to a solid phase by means of a linking polymer which is covalently connected to the molecule at the distal end of the polymer and to the solid phase at the proximal end of the polymer the improvement which comprises: a polyalkylene ether linking polymer selected from
$XC_2H_3R(OC_2H_3R)_nOC_2H_3RX$
wherein n is 5 to 200, R is selected from the group consisting of hydrogen or alkyl and X is selected from the group consisting of $-NH_2$, $-NHCONHNH_2$, $-NHCO(CH_2)_pSH$, $-OCO(CH_2)_pSH$, wherein p is 1 to 3, and a

halogen selected from the group consisting of chlorine and bromine and from
$XCH_2(OC_2H_3R)_nOCH_2X$
wherein n is 5 to 200, R is H or alkyl and X is selected from the group consisting of $-CO_2H$ or $-CONHNH_2$ which is covalently linked by reaction with one of X with the solid phase and with the other of X can be covalently linked with the biologically useful molecule or contains the biologically useful molecule, wherein the linking polymer is attached to the exposed surface of the solid phase in a density between about 10 to 100 nanomoles per square meter of the exposed surface area of the solid phase.

-9- In a device for attaching a biologically useful molecule to a solid phase by means of a linking polymer which is covalently connected to the molecule at the distal end of the polymer and to the solid phase at the proximal end of the polymer the improvement which comprises:
a linking polymer of the formula

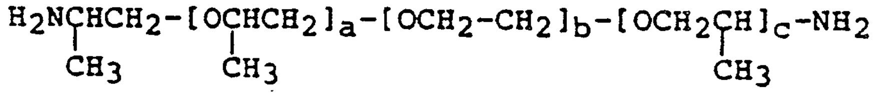

where a plus c are 2.5
and b is 8.5 and 131.5 and wherein the length of the linking molecule is 50 and 650 Angstroms.

-10- The device of Claim 7 wherein the biologically useful molecule is linked to the linking polymer.

-11- The device of Claim 8 wherein the biologically useful molecule is linked to the linking polymer.

-12- The device of Claim 9 wherein the biologically useful molecule is linked to the linking polymer.

-13- A process for attaching a linking polymer to a solid phase to provide a support for a biologically useful molecule which comprises:

(a) providing a linking polymer which is a polyalkylene ether linking polymer selected from
$XC_2H_3R(OC_2H_3R)_nOC_2H_3RX$
wherein n is 5 to 200, R is selected from the group consisting of hydrogen or alkyl and X is selected from the group consisting of $-NH_2$, $-NHCONHNH_2$, $-NHCO(CH_2)_pSH$, $-OCO(CH_2)_pSH$, wherein p is 1 to 3, and a halogen selected from the group consisting of chlorine and bromine and
$XCH_2(OC_2H_3R)_nOCH_2X$
wherein n is 5 to 200, R is H or alkyl and X is selected from the group consisting of $-CO_2H$ or $-CONHNH_2$ which is covalently linked by reaction with one of X with the solid phase and with the other of X which can be covalently linked with the biologically useful molecule or contains the biologically useful molecule, wherein the linking polymer is attached to the exposed surface of the solid phase in a density between about 10 to 100 nanomoles per square meter of the exposed surface area of the solid phase;

(b) reacting a solid phase with limited amounts of an activating agent in a solvent which is non-reactive with the activating agent to provide exposed groups which are reactive with X at one end of the linking polymer, wherein the exposed groups are present in a density of less than 100 nanomoles per square meter; and

(c) reacting the linking polymer with the solid phase in a solvent for the linking polymer, wherein the linking polymer is attached to the solid phase in a density of less than about 100 nanomoles per square meter.

-14- The process of Claim 13 wherein the linking polymer is

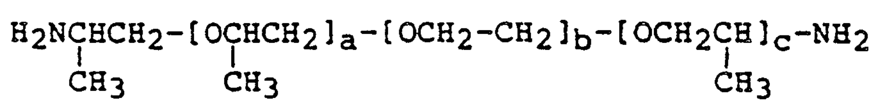

where a plus c are 2.5
and b is between 8.5 and 131.5 and wherein the length of the linking molecule is about 50 to 650 Angstroms.

-15- The process of Claim 13 wherein the solid phase is nylon and the exposed group is produced by reacting the nylon with triethoxonium fluoroborate in an anhydrous organic solvent as the solvent and wherein X is $-NH_2$.

-16- The process of Claim 13 wherein the solid phase is aminopolystyrene reacted with 1,1′-carbonyl diimidazole and then the linking polymer in an anhydrous polar solvent as the solvent and wherein X is $-NH_2$.

-17- The process of Claim 13 wherein the linking polymer is covalently connected to an enzyme as the biologically useful molecule at a distal end.

-18- The process of Claim 13 wherein the useful molecule comprises enzymes covalently linked together and wherein at least one of which is covalently connected to a distal end of the linking molecule.

-19- The process of Claim 18 wherein the enzymes are a peroxidase and a glucose oxidase covalently linked together as a cluster of the two enzymes by reaction with adipic dihydrazide.

-20- The process of Claim 19 wherein low molecular weight impurities are removed from the cluster by dialization prior to being covalently linked with the linking polymer.